# EUROPEAN PATENT APPLICATION

(11) **EP 3 202 375 A1**
(43) Date of publication of application: **09.08.2017**
(21) Application number: 17152287.3
(22) Date of filing: 19.01.2017
(51) Int. Cl.: A61F 5/01, A61F 5/058

(54) **ORTHOPAEDIC APPLIANCE FOR USE IN TREATING FRACTURED CLAVICLES**

(30) Priority: 21.01.2016 IT UB20160371
(71) Applicant: Tecnoway s.r.l., 61022 Morciola di Colbordolo (PU) (IT)
(72) Inventor: SCATASSA, Ettore, 61022 Località Morciola - Vallefoglia (IT)
(74) Representative: Tiburzi, Andrea

(57) **Abstract**

The present invention concerns a brace (1) for a bone district of a user (U) having at least a bone to be recomposed or to be maintained, as a clavicle (C) and the like, comprising a support (2), having an inner surface (211), intended to adhere to said bone district of said user (U), characterized in that said support (2) comprises at least one first (23) and one second (24) padding, arranged on said inner surface (211), said first (23) and second (24) padding realizing a longitudinal housing (25), in which the bone (C) of said bone district to be recomposed is intended to be positioned.

## Description

The present invention relates to an improved brace for a bone district, particularly for the clavicle.

More specifically, the invention concerns a brace of the above type, studied and realized in particular for the clavicle, capable of allowing an optimal positioning thereof with respect to the clavicle, thus allowing a perfect fixing of the clavicle itself.

In the following, the description will be directed to the clavicle conservation, but it is clear that the same should not be considered limited to this specific use.

The clavicle is a bone of the shoulder, it is "S" shaped and it has two joint ends. The particular shape of this bone is due to two concavities, one medial (rear) and one side (front). This conformation makes such bone particularly difficult to block, in case of fracture or damage in general.

As it is well known there are currently several types of clavicle braces, which generally have the problem of adapting to the bone shape, which is as said irregular, and size, depending on the age of the patient.

More specifically, for the conservative treatment of the dislocation and sub-dislocation of the acromioclavicular-clavicular or to treat the fracture of the distal end of the clavicle, different clavicle immobilizations are required.

The braces according to the prior art have specific forms, suitable for specific conservative treatments. This implies, therefore, according to the different types of fractures or dislocations, specific different braces are necessary.

It appears obvious that this procedure is expensive in economic terms, since the clavicle braces manufacturers must provide different types of braces, suitable for specific uses.

Patent applications WO 2016/001802A1, US 2007/106187 and NL 1039802C are also part of the known prior art.

The aforementioned patent applications provide all paddings or inflatable bladders designed to allow the user to wear the braces in the best way. However, the above listed patent rights do not provide any way to fix a specific bone district, such as a clavicular or the like.

In the light of the above, it is, therefore, object of the present invention to propose a brace capable of appropriately adapting to the various types of dislocation fractures, particularly designed to fit optimally to the clavicle, but which can be used for any other body part that needs proper immobilization.

It is therefore specific object of the present invention a brace for a bone district of a user having at least a bone to be reassemble or to be conserved, as a clavicle and the like, comprising a support, having an inner surface, intended to adhere to said bone district of said user, characterized in that said support comprises at least one first and one second padding, arranged on said inner surface, said first and second padding realizing a longitudinal housing, in which the bone of said bone district to be reassembled is intended to be positioned.

Always according to the invention, said first and/or second padding could be made by a material with a preset degree of rigidity, such as rubber, foam rubber, silicone and the like, so as to fit the shape and the physique of said user, in case of a preset pressure.

Still according to the invention, said first padding could be constituted by an inflatable bladder and/or said second padding could constituted by an inflatable bladder.

Advantageously according to the invention, said brace could comprise at least one duct connected to the bladder of said first padding and/or to the bladder of said second padding, and it could further comprise at least one one-way valve, in which a pump is connectable to said at least one duct to inflate and deflate said bladder of said first padding and/or said bladder of said second padding by said at least one one-way valve.

Preferably, according to the invention, said brace could comprise a switch for selectively inflate or deflate said bladder of said first padding and/or said bladder of said second padding.

Further according to the invention, said brace could comprise a first and a second duct, each one connected to a respective bladder of said first padding and/or bladder of said second padding, on each of said ducts a one-way valve being arranged, for inflating and deflating said bladder of said first padding and/or said bladder of said second padding.

Always according to the invention, said second padding could have three lobes, wherein two of said lobes contribute, with said first bladder, to form said longitudinal housing, and said third lobe is intended to be placed in correspondence of the scapula-humeral girdle, for preserving the clavicular coracoacromial ligament of said user wearing said brace.

Still according to the invention, said support could comprise a main support band.

Advantageously according to the invention, said brace could comprise a pair of positioning strips, each one having the first end fixed to said main support band and the second end fixed to the second end of the other positioning strip, said positioning strips and said main support band forming between them an opening or slot, such that, when said brace is worn for positioning a clavicle, the upper portion of the deltoid of the shoulder corresponding to said clavicle inserts in said opening or slot.

Preferably, according to the invention, a coupling member could be fixed in the coupling point of the second end of each positioning strip, and said brace could comprise a support for the arm and suspenders for connecting said coupling member and said support for the arm and said main support band and said support for the arm.

Further according to the invention, said brace could comprise a further support strip, fixed to said main support band so as to be arranged with respect to the same, so that said support is substantially "T" shaped, said support being positioned on the shoulder, and it could comprise a support for the arm and suspenders for connecting said main support band, said further support strip and said support for the arm.

Always according to the invention, said first padding could be arranged upward with respect to said second padding.

The present invention will be now described, for illustrative but not limitative purposes, according to its preferred embodiments, with particular reference to the figures of the enclosed drawings, wherein:
figure 1 shows a user wearing a first embodiment of a brace for clavicle according to the present invention;
figure 2 shows a transparent view of the brace of figure 1;
figure 3 shows a perspective view of the brace according to the present invention while immobilizes a clavicle;
figure 4 shows a further perspective view of the brace according to figure 3;
figure 5 shows a pump for inflating the bladders of the brace according to figures 3 or 4;
figure 6 shows a bottom perspective view of a second embodiment of a brace for clavicle according to the present invention;
figure 7 shows a top perspective view of the brace according to figure 6; and
figure 8 shows a worn brace according to figure 6.

In the various figures, similar parts will be indicated by the same reference numbers.

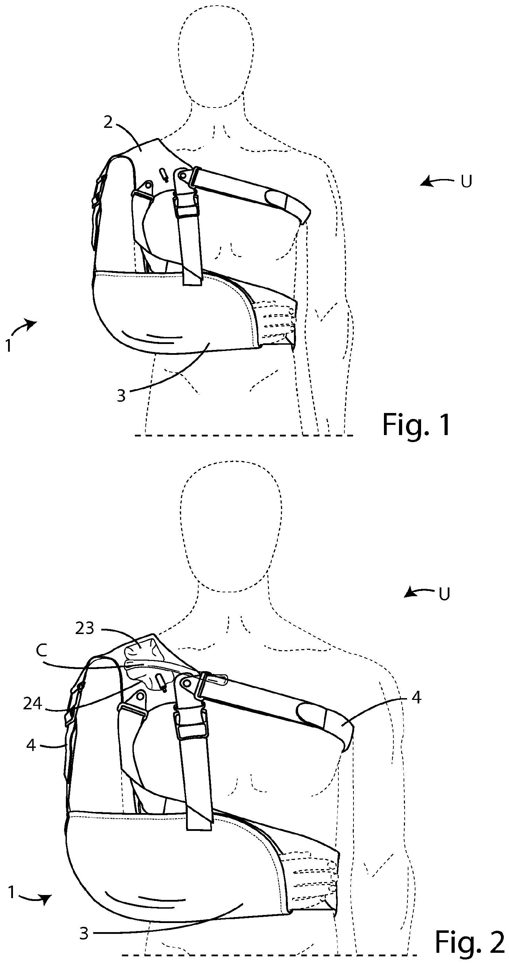

Referring to figures 1 and 2, it is observed a user U to which a brace for clavicle 1 according to the present invention is applied.

The brace 1 comprises essentially a support for the shoulder 2, a support for the arm 3 and suspenders 4, which connect in an adjustable way, said support for the shoulder 2 and said support for the arm 3.

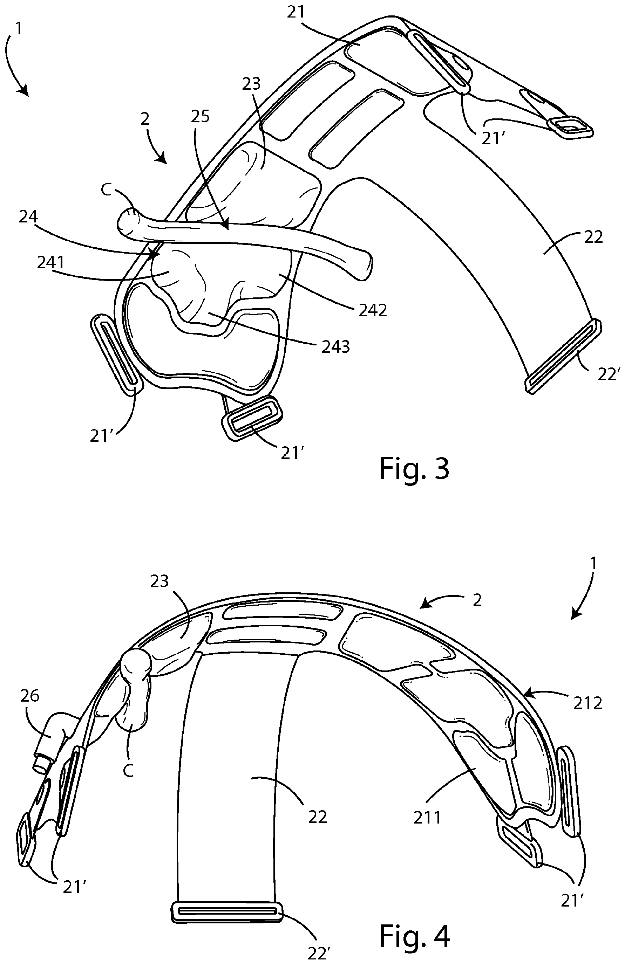

Examining in detail the support for the shoulder 2, and making reference also to figures 3 and 4, it is observed that it includes a main support band 21 and a further support band 22, arranged each other in such a way that said support for the shoulder 2 is substantially "T" shaped.

Said main support band 21 is adapted to bind the shoulder of the user U. Said main support band 21 has an inner surface 211, intended to adhere with said shoulder, and an outer surface 212.

At the ends and/or at the edges of said main support band 21 and of said further support band 22 the rings 21' and 22' are fixed for the connection of suspenders 4 to said support for the shoulder 2.

Said support for the shoulder 2 also comprises a first and a second padding, indicated respectively by the numerical references 23 and 24, arranged on said inner surface 211, between which a space or housing 25, having a longitudinal shape, is formed, wherein the bone to be reconstructed is intended to be positioned, which in this case is the clavicle C.

In particular, when said brace 1 is worn by a user U said first padding 23 is arranged upward with respect to said second padding 24.

Moreover, said second padding 24 has a shape with three lobes, or trilobed, each one indicated by the reference numbers 241, 242 and 243.

Two of said lobes 241 and 242 combine each other together with said first padding 23to form said housing 25.

The third lobe 243 is intended, however, to be located at the scapula-humeral girdle, for the conservation of the clavicular coracoacromial ligament of the user U, who wears the brace 1.

Said paddings can be made in general by means of a semi-rigid material, deformable or with a predetermined degree of rigidity, such as rubber, foam rubber, silicone and the like, and in any case so as to fit the shape and the user's U constitution, in case of a preset pressure.

As it can be seen from figures 3 and 4 shown, also, the housing 25 formed between said first 23 and second padding 24 is oriented slightly obliquely with respect to the extension direction of said main support band 21, so as to allow a better positioning, in this case, of said clavicle C between said first 23 and second 24 padding.

In a preferred embodiment (shown in particular in figures 3 and 4), said first 23 and second 24 padding are constituted by two inflatable bladders.

Connected to said first 23 and second 24 inflatable bladder, a conduit 26 is also provided, within which a one-way valve (not shown in the figures), that can be opened if necessary, and possibly a switch (not shown in the figures), to selectively inflate or deflate each bladder 23 or 24, are provided.

The operation of the brace 1 described above is as follows.

When said brace 1 is worn by a user U, it is placed as any other brace, intended, for example, for the conservative treatment of the dislocation and sub-dislocation of the acromion-clavicular joint or to treat a fracture of the distal end of a clavicle C. As unique feature, the bone region to be treated, which in this case is, as mentioned, a clavicle C, is arranged between said first 23 and second 24 padding.

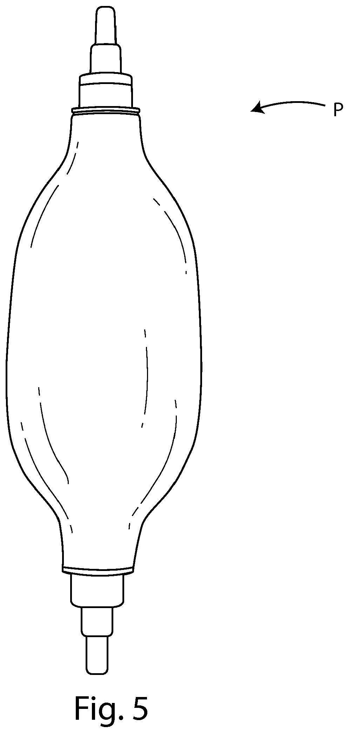

Then, in the embodiment, in which the paddings 23 and 24 are inflatable bladders, a suitable pump P, shown by way of example in figure 5, is connected to said conduit 26 and, by means of said switch, for example, said first padding 23 is inflated.

Once said first padding 23 is sufficiently inflated, according to the user's U physique and physical conformation, it can be possible again to act on said switch, to inflate said second padding 24.

In this way, said first padding 23 and said two lobes 241 and 242 of said second padding 24 allow to keep and retain the clavicle between them arranged in said housing 25.

Instead, the third lobe 243 of said second padding 24 arranges and fits in correspondence of the scapula-humeral girdle, possibly filling the physiological cavity of the user, according to the physique and the specific anatomy of the latter.

This conformation of the padding 23 and 24, allows to optimally adapt the form of the brace 1 to the bone to be conserved, which, in this case, as mentioned, is a clavicle C, according to both the user's U conformation and physique, and the type of fracture or dislocation the bone district is concerned of.

To deflate each padding 23 or 24, it is sufficient to open said one-way valve and act on the switch, deflating a padding at a time. It is possible then to proceed with the removal of the brace 1.

In addition, according to a further embodiment, it is possible to provide two separate ducts 26, each one connected to the bladder of the padding 23 or 24 and having a respective one-way valve to independently inflate and deflate said paddings 23 and 24.

According to a further embodiment, providing that the three lobes 241, 242 and 243 of the bladder of said second padding 24 are independently inflated is possible, to better adapt the brace 1.

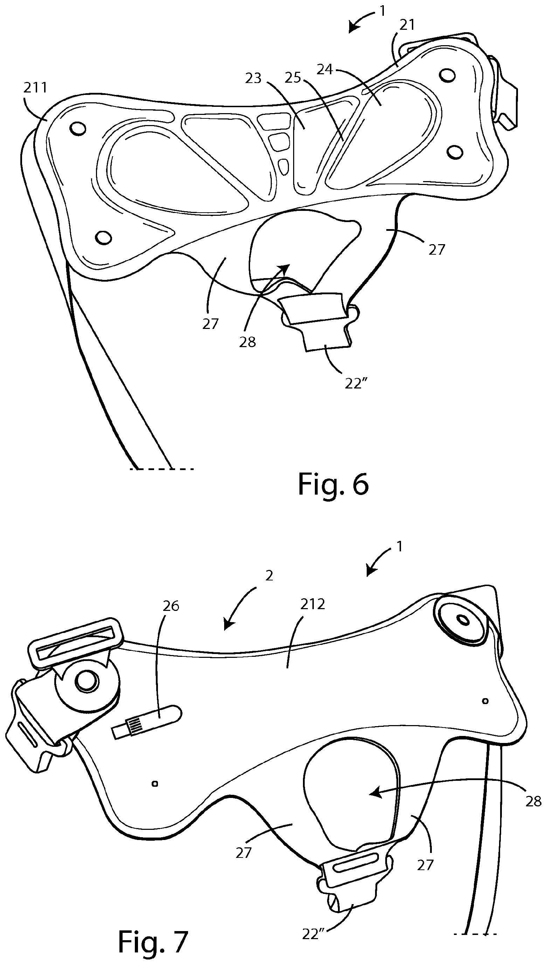

Referring now to figures 6-8, a second embodiment of the clavicle brace 1 according to the present invention is observed.

In the clavicle brace 1 it is observed that the second padding 24 is not a three-lobed shape. However, said second embodiment can provide the three-lobed shape, similar to that shown in figure 3.

The clavicle brace 1 comprises, in lieu of the further support band 22, a pair of positioning strips 27, each one having a first end fixed to said main support band 21 and the second end fixed to the second end of the other positioning strip 27. In the coupling point of the second end of each one of the positioning strip 27 a coupling member 22" is fixed, to which in its turn is fixable to a further a strip adjustable in length and coupleable to the support for the arm 3.

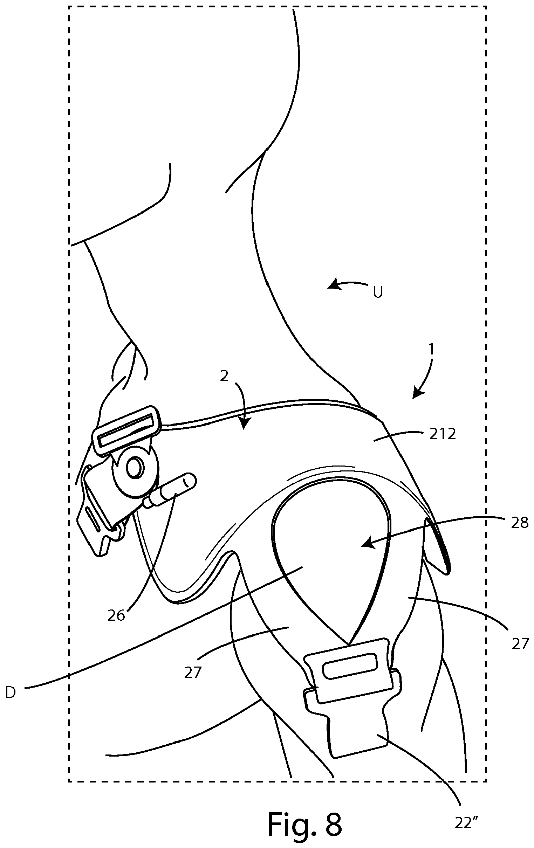

Said positioning strips 27 and said main support band 21 form between them an opening or slot 28, such that, when said brace 1 is worn (see in particular figure 8), the upper portion of the deltoid D of the shoulder corresponding to the lesioned clavicle, fits into said opening or slot 28.

In this way, there is a fixing synergistic effect between said positioning strips 27 and said first and second padding 23 and 24, immobilizing the clavicle, preventing any stress of the same.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Brace (1) for a bone district of a user (U) having at least a bone to be reassemble or to be conserved, as a clavicle (C) and the like, comprising
a support (2), having an inner surface (211), intended to adhere to said bone district of said user (U),
**characterized in that** said support (2) comprises at least one first (23) and one second (24) padding, arranged on said inner surface (211), said first (23) and second (24) padding realizing a longitudinal housing (25), in which the bone (C) of said bone district to be reassembled is intended to be positioned.

2. Brace (1) according to claim 1, **characterized in that** said first (23) and/or second (24) padding can be made by a material with a preset degree of rigidity, such as rubber, foam rubber, silicone and the like, so as to fit the shape and the physique of said user (U), in case of a preset pressure.

3. Brace (1) according to any one of the preceding claims, **characterized**
**in that** said first padding (23) is constituted by an inflatable bladder and/or
**in that** said second padding (24) is constituted by an inflatable bladder.

4. Brace (1) according to claim 3, **characterized**
**in that** it comprises at least one duct (26) connected to the bladder of said first padding (23) and/or to the bladder of said second padding (24), and
**in that** it further comprises at least one one-way valve, in which a pump (P) is connectable to said at least one duct (26) to inflate and deflate said bladder of said first padding (23) and/or said bladder of said second padding (24) by said at least one one-way valve.

5. Brace (1) according to any one of claims 3 or 4, **characterized in that** it comprises a switch for selectively inflate or deflate said bladder of said first padding (23) and/or said bladder of said second padding (24).

6. Brace (1) according to any one of claims 4 or 5, **characterized in that** it comprises a first and a second duct, each one connected to a respective bladder of said first padding (23) and/or bladder of said second padding (24), on each of said ducts a one-way valve being arranged, for inflating and deflating said bladder of said first padding (23) and/or said bladder of said second padding (24).

7. Brace (1) according to any one of the preceding claims, **characterized in that** said second padding (24) has three lobes (241, 242, 243), wherein two of said lobes (241, 242) contribute, with said first bladder (23), to form said longitudinal housing (25), and said third lobe (243) is intended to be placed in correspondence of the scapula-humeral girdle, for preserving the clavicular coracoacromial ligament of said user (U) wearing said brace (1).

8. Brace (1) according to any one of the preceding claims, **characterized in that** said support (2) comprises a main support band (21 ).

9. Brace (1) according to claim 8, **characterized**
**in that** it comprises a pair of positioning strips (27), each one having the first end fixed to said main support band (21) and the second end fixed to the second end of the other positioning strip (27),
said positioning strips (27) and said main support band (21) forming between them an opening or slot (28), such that, when said brace is worn for positioning a clavicle, the upper portion of the deltoid (D) of the shoulder corresponding to said clavicle inserts in said opening or slot (28).

10. Brace (1) according to claim 9, **characterized**
**in that** a coupling member (22") is fixed in the coupling point of the second end of each positioning strip (27), and
**in that** it comprises a support for the arm (3) and suspenders (4) for connecting said coupling member (22") and said support for the arm (3) and said main support band (21) and said support for the arm (3).

11. Brace (1) according to claim 8, **characterized in that** it comprises a further support strip (22), fixed to said main support band (21) so as to be arranged with respect to the same, so that said support (2) is substantially "T" shaped, said support (2) being positioned on the shoulder, and
**in that** it comprises a support for the arm (3) and suspenders (4) for connecting said main support band (21), said further support strip (22) and said support for the arm (3).

12. Brace (1) according to any one of the preceding claims, **characterized in that** said first padding (23) is arranged upward with respect to said second padding (24).
